(19) Europäisches Patentamt
European Patent Office
Office européen des brevets

(11) Publication number: 0 053 584
B1

(12) EUROPEAN PATENT SPECIFICATION

(45) Date of publication of patent specification: 17.04.85

(51) Int. Cl.4: C 07 D 207/09, C 07 B 57/00

(21) Application number: 81830221.8

(22) Date of filing: 13.11.81

# (54) Raceme sulpiride resolution process.

(30) Priority: 27.11.80 IT 2627580

(43) Date of publication of application:
09.06.82 Bulletin 82/23

(45) Publication of the grant of the patent:
17.04.85 Bulletin 85/16

(84) Designated Contracting States:
BE DE FR GB LU NL

(56) References cited:
FR-A-2 314 178
GB-A-1 555 890
GB-A-2 014 990

CHEMICAL ABSTRACTS, vol. 89, no. 25, 18-12-1978, page 577, abstract 215217z Columbus, Ohio, US

(73) Proprietor: RAVIZZA S.p.A.
35, Via Europa
I-20053 Muggio' (Milano) (IT)

(72) Inventor: Mauri, Francesco
Via A. Boito, 65
Monza (Milano) (IT)

(74) Representative: Gervasi, Gemma et al
Studio Brevetti e Marchi NOTARBARTOLO & GERVASI 33, Viale Bianca Maria
I-20122 Milano (IT)

EP 0 053 584 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the resolution of raceme sulpiride with high yields of both levo-sulpiride and dextro-sulpiride of pharmaceutical purity.

Sulpiride or N-ethyl-2(2-methoxy-5-sulphamido-benzamidomethyl)pyrrolidine of formula

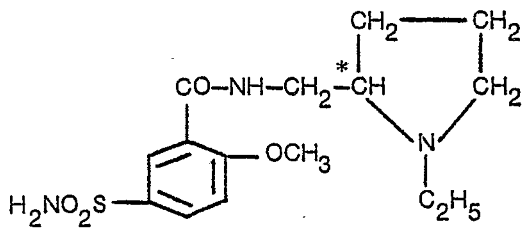

is known to be therapeutically active compound particularly useful in the neurological and psychiatric field as an antipsychotic.

It is also known that sulpiride, which contains one asymmetric carbon atom (indicated by an asterisk in the formula), is in reality an equimolecular mixture of two optically active compounds of which the levo-sulpiride is much more effective as a psychiatric drug than the dextro-sulpiride. Many attempts have been made up to the present time to isolate the more active levo-sulpiride from the raceme mixture, all consistently with negative results.

A stereospecific synthesis was therefore devised (French Patent FR—A 2 417 498).

This synthesis, which uses proline as its starting substance, can be used only for the production of levo-sulpiride from l-proline, because d-proline, which would be necessary for preparing dextro-sulpiride by the same method, is a product not available commercially and very costly to prepare.

It has now been found that dextro-sulpiride, which is practically ineffective as an antipsychotic, has a quite unexpected strong therapeutic activity towards forms of behaviour decadence in senescence. This is the basis for the need to find an industrial method for its production.

A method for the resolution of raceme sulpiride has been unexpectedly found which enables both optically pure levo-sulpiride and optically pure dextro-sulpiride to be obtained with very high yields. The conversions are also high, and the unconverted raceme sulpiride can be recovered practically completely. The method is consequently industrially economical for the production of levo- and dextro-sulpiride, a further factor being that there is no consumption of costly reactants or solvents.

The new method consists essentially of salifying raceme sulpiride with an equimolecular quantity of L(−)pyroglutamic acid to give a salt of formula:

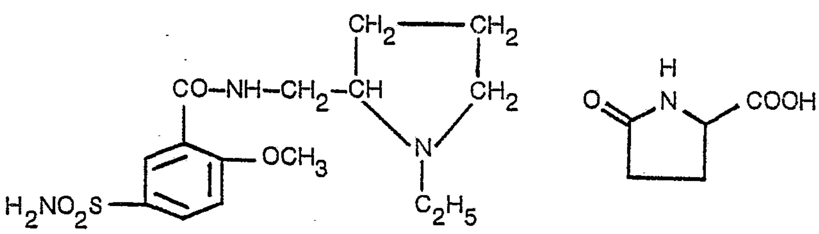

in 95 vol % ethanol, the solution being heated to its boiling point.

On cooling the ethanol solution containing the mixture of L(−)pyroglutamate of levo-sulpiride and L(−)pyroglutamate of dextro-sulpiride to 20°C, the L(−)pyroglutamate of levo-sulpiride separates in crystalline form and is filtered off, the L(−)pyroglutamate of dextro-sulpiride being obtained by evaporating the cold residual solution.

The free levo- and dextro-sulpiride are recovered from said salts by hydrolysis with $NH_4OH$.

L(-)pyroglutamic acid is recovered from the alkaline mother liquors from which the levo-sulpiride and dextro-sulpiride were precipitated, and is recycled.

The raceme sulpiride present as an impurity in both the separated levo-sulpiride and dextro-sulpiride is recovered from these products by fractional crystallisation of the relative hydrochlorides.

In order to facilitate reproduction of the process according to the present invention, one example is described hereinafter, and contains all the operating details.

It is however apparent that these details can be varied without altering the inventive idea and thus leaving its scope.

Example

| | |
|---|---|
| Raceme sulpiride | 46 g (0.135 M) |
| L(-)pyroglutamic acid | 17.5 g (0.135) M) |
| 95 vol % ethanol | 200 ml |

The mixture of these substances is heated to boiling point until completely dissolved, the solution then being cooled to 20°C and filtered.

The crystalline precipitate when dried weighs 32.5 g. The mother liquors when evaporated to dryness leave a vitreous residue weighing 31 g.

The two solids are dissolved separately each in 100 ml of deionised water, and the solutions obtained are each treated with ammonium hydroxide at 70°C until pH 10 is attained. They are then cooled and filtered.

By hydrolysis of the crystalline product solution, 23 g of product are obtained which after drying has a $[\alpha]_D^{20}$ of −42.5° (5% DMF).

Under the same conditions, the solution obtained from the vitreous residue gives 21.5 g of product which after drying has a $[\alpha]_D^{20}$ of +42.5° (5% DMF).

The mother liquors from the two precipitations are combined, evaporated to low volume, taken up in deionised water and eluted through a column of a strongly acid resin, for example Amberlite IR 120, on the basis of about 1 g of product per 10 ml of activated resin.

The elution is carried out with deionised water, and various 50 ml fractions are collected.

The first three fractions contain the L(−)pyroglutamic acid, and by evaporating these to dryness 16 g of acid are recovered, which can be recycled.

The two optically active sulpirides still impure with raceme product are each dissolved under hot conditions in a stoichiometric quantity of 1N HCl.

They are allowed to crystallise at 20°C and are filtered. The hydrochlorides obtained by crystallisation are combined, dissolved in 150 ml of water at 70°C, the solution adjusted to pH 10 with ammonium hydroxide, cooled, filtered and the product dried.

The product obtained (18 g) is constituted by practically raceme sulpiride, and is recycled.

The hydrochloride liquors are heated to 70°C, alkalised with ammonium hydroxide to pH 10, and cooled.

When filtered and dried, the products obtained have the following characteristics;

— 12.5 g of L(−)sulpiride; $[\alpha]_D^{20}$ −65° (5% DMF);
M.P. 188—190°C

— 12.5 g of D(+)sulpiride; $[\alpha]_D^{20}$ +65° (5% DMF);
M.P. 188—190°C.

The following are substantially obtained from 46 g of raceme sulpiride:

L(−)sulpiride 12.5 g
D(+)sulpiride 12.5 g
Raceme sulpiride 18 g
Efficiency 93% with 54% conversion.
16 g of acid for recycling are recovered from 17.5 g of L(−)pyroglutamic acid.

## Claims

1. A process for preparing optically pure levo-sulpiride and dextro-sulpiride, characterised in that the raceme sulpiride is salified with an equimolecular quantity of L(−)pyroglutamic acid in 95 vol % ethanol at boiling point to form a solution of a mixture of salts of formula

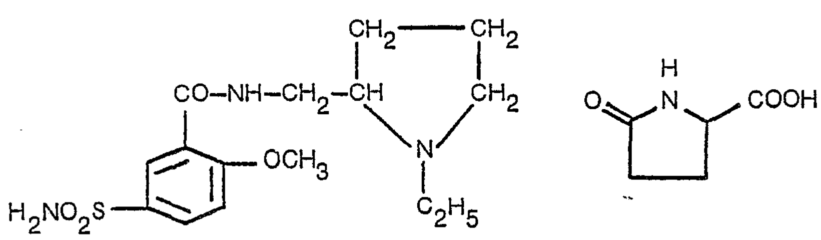

from which the crystalline L(−)pyroglutamate of levo-sulpiride is separated by cooling to 20°C, whereas the L(−)pyroglutamate of dextro-sulpiride is obtained by evaporating the residual ethanol solution, the two optically active bases being obtained by alkaline hydrolysis of said pyroglutamates, in separate aqueous solutions.

2. A process as claimed in claim 1, wherein the alkaline hydrolysis is carried out with $NH_4OH$.

3. A process as claimed in claim 1, wherein the levo-sulpiride and dextro-sulpiride are further purified from the residual raceme product by fractional crystallisation of the relative hydrochlorides.

4. A process as claimed in claim 1, wherein the L(−)pyroglutamic acid is recovered by eluting the alkaline mother liquors through a strongly acid resin.

**Patentansprüche**

1. Verfahren zum Herstellen von optisch reinem Laevo-sulpirid und Dextro-sulpirid, dadurch gekennzeichnet, daß das racemische Sulpirid mit einem äquimolaren Anteil L-(−)-Pyroglutaminsäure in 95 Vol.% Äthanol beim Siedepunkt in die Salzform überführt wird, wobei eine Lösung einer Mischung von Salzen der Formel

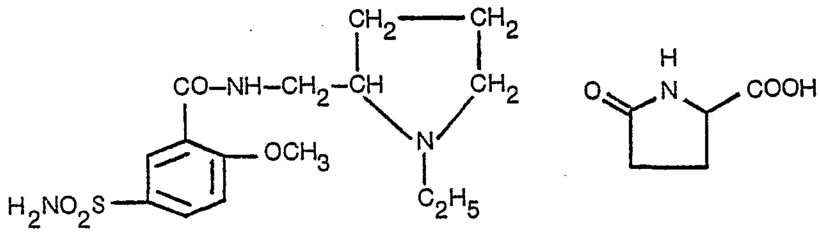

erhalten wird, aus der das kristalline L-(−)-Pyroglutamat von Laevo-sulpirid durch Kühlen auf 20°C abgetrennt wird, währen das L-(−)-Pyroglutamat von Dextro-sulpirid durch Eindampfen der restlichen Äthanollösung erhalten wird, wobei die beiden optisch aktiven Basen durch alkalische Hydrolyse dieser Pyroglutamate in getrennten wässerigen Lösungen erhalten werden.

2. Verfahren, wie in Anspruch 1 beansprucht, wobei die alkalische Hydrolyse mit $NH_4OH$ durchgeführt wird.

3. Verfahren, wie in Anspruch 1 beansprucht, wobei das Laevo-sulpirid und das Dextro-sulpirid von dem verbleibenden racemischen Produkt durch fraktionierte Kristallisation der betreffenden Hydrochloride weiter gereinigt werden.

4. Verfahren, wie in Anspruch 1 beansprucht, wobei die L-(−)-Pyroglutaminsäure durch Eluieren der alkalischen Mutterlaugen durch ein stark saures Harz gewonnen wird.

**Revendications**

1. Procédé pour préparer du lévo-sulpiride et du dextro-sulpiride à l'état d'isomères optiques purs, caractérisé en ce que l'on salifie le sulpiride racémique par une quantité équimoléculaire d'acide L(−)-pyroglutamique dans l'éthanol à 95% en volume au point d'ébullition, formant une solution d'un mélange de sels de formule

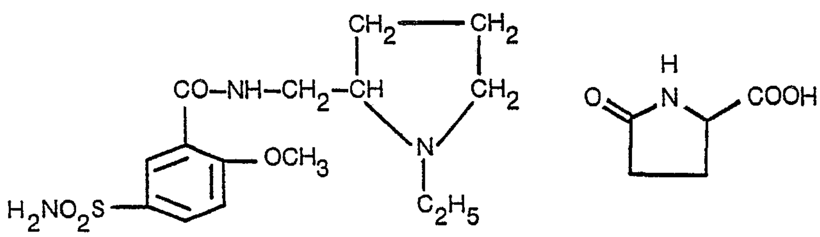

d'où l'on sépare le L(−)-pyroglutamate du lévo-sulpiride à l'état cristallisé par refroidissement à 20°C, après quoi on obtient le L(−)-pyroglutamate du dextro-sulpiride en évaporant la solution éthanolique résiduelle, les deux bases étant obtenues à l'état d'isomères optiques par hydrolyse alcaline de ces pyroglutamates dans des solutions aqueuses séparées.

2. Procédé selon la revendication 1, dans lequel l'hydrolyse alcaline est effectuée à l'aide de $NH_4OH$.

3. Procédé selon la revendication 1, dans lequel le lévo-sulpiride et le dextro-sulpiride sont en outre purifiés par élimination du racémique résiduel par cristallisation fractionnée des chlorhydrates correspondants.

4. Un procédé selon la revendication 1, dans lequel on récupère l'acide L(−)-pyroglutamate en éluant les liqueurs-mères alcalines sur une résine fortement acide.